# EUROPEAN PATENT SPECIFICATION

(11) **EP 2 964 185 B1**
(45) Date of publication and mention of the grant of the patent: **10.11.2021**
(21) Application number: 14760660.2
(22) Date of filing: 05.03.2014
(51) Int. Cl.: A61K 31/137, A61K 31/405, A61K 31/417, A61K 31/4402, A61K 31/135, A61K 31/4045, A61K 31/4415, A61K 31/455, A61K 33/06, A61K 8/67, A61K 45/06, A23L 33/10, A23L 33/15, A23L 33/175, A61K 36/84, A61P 11/00

(54) **PREPARATIONS FOR THE TREATMENT OF SLEEP-RELATED RESPIRATORY DISORDERS**
ZUBEREITUNGEN ZUR BEHANDLUNG SCHLAFBEZOGENER ATMUNGSSTÖRUNGEN
PRÉPARATIONS POUR LE TRAITEMENT DE TROUBLES RESPIRATOIRES LIÉS AU SOMMEIL

(30) Priority: 05.03.2013 US 201361773045 P
(43) Date of publication of application: 13.01.2016
(73) Proprietor: Requis Pharmaceuticals Inc., West Chester, PA 19382 (US)
(72) Inventor: KNUTSEN, Lars Jacob, Stray, West Chester, PA 19382-7101 (US); HAND, James, M., Collegeville, PA 19426 (US)
(74) Representative: Manaton, Ross Timothy
(86) International application number: PCT/US2014/020542
(87) International publication number: WO 2014/138162

(56) References cited:
- WO-A1-98/51309
- WO-A1-2007/092333
- WO-A1-2012/170883
- WO-A1-2012/170883
- WO-A2-02/056876
- US-A1- 2004 157 813
- US-A1- 2006 039 867
- US-A1- 2006 130 833
- US-A1- 2011 245 323
- US-A1- 2012 195 968
- US-B1- 6 258 814
- None

## Description

### FIELD OF THE INVENTION

The present invention is directed to combinations of a sedating antihistamine and certain indole-based dietary supplements, minerals and vitamins, in relation to their use for improving the health of human subjects, by the treatment of sleep-related respiratory disorders, which are characterized by abnormal breathing during sleep. These include snoring, sleep disordered breathing, sleep apneas, upper airway resistance syndrome, obstructive sleep apnea, central sleep apnea, catathrenia and obesity hypoventilation syndrome.

### BACKGROUND OF THE INVENTION

Sleep-related respiratory disorders represent a series of conditions featuring abnormal breathing during periods set aside for sleep, the most prominent of which are snoring and sleep disordered breathing. Sleep-Disordered breathing describes a group of disorders characterized by abnormalities of respiratory pattern (pauses in breathing) or in the quantity of ventilation during sleep.

Sleep related respiratory disorders include a continuum of conditions ranging from primary snoring through obstructive sleep apnea/hypopnea (OSAH) syndrome. They are omnipresent in our society and are gaining recognition for their effects on quality of life as well as their direct relationship with well-accepted diseases such as hypertension, stroke and congestive heart failure. OSAH is characterized by repetitive episodes of upper airway obstruction that occur during sleep and are usually associated with reductions in blood oxygen saturation and frequently associated with cortical electroencephalogram arousal activity. The term Pickwickian syndrome was originally applied to those patients who had sleepiness, obesity, and evidence of right heart failure. Some of these patients probably had central alveolar hypoventilation syndrome or obesity hypoventilation syndrome rather than typical OSAH, as we now know it. The Wisconsin sleep cohort study of patients without obvious barriers to health care access estimated that 93% of women and 82% of men with moderate-to-severe sleep apnea were undiagnosed (Young, T. Rationale, Design, and Findings from the Wisconsin Sleep Cohort Study: Toward Understanding the Total Societal Burden of Sleep-Disordered Breathing. Sleep Med. Clin., 2009, 4, 37-46). Significant cardiovascular morbidity (including systemic hypertension and congestive heart failure) and increased mortality rates have been associated with obstructive sleep apnea and hypopnea. While large-scale studies of the prevalence of sleep-disordered breathing in children are lacking, a 1988 study estimated 5-6% and raised concerns about the impact of the obesity epidemic on sleep in children. (Guilleminault C.; Quera-Salva, M.A., Powell, N. et al., Nocturnal asthma: snoring, small pharynx and nasal CPAP. Eur. Respir. J., 1988, 1, 902 - 907).

Sleep apneas consist of several sub-classes of apneas, which include Central Apnea Syndromes, Cheyne-Stokes Respiration, Obstructive Sleep Apnea Syndrome, Upper Airway Resistance Syndrome, and Alveolar Hypoventilation Syndrome (Tsara, V.; Amfilochiou, A.; Papagrigorakis, M.J.; Georgopoulos, D.; Liolios, E. Guidelines for Diagnosis and Treatment of Sleep-related Breathing Disorders in Adults and Children. Definition and classification of sleep related breathing disorders in adults. Different types and indications for sleep studies (Part 1), Hippokratia, 2009, 13, 187-191).

Obstructive sleep apnea is a condition characterized by repetitive obstruction of the upper airway often resulting in oxygen desaturation and arousals from sleep. The classic daytime manifestation is excessive sleepiness but other symptoms such as unrefreshing sleep, poor concentration and fatigue are commonly reported. Over the past 30 years many types of abnormal breathing during sleep have been described that are related to, but not accurately described as apneas. Partial airway obstruction can lead to a reduction in tidal volume, referred to as a hypopnea, with the same consequences as an apnea.

The sleep related respiratory disorder known commonly as snoring occurs when the flow of air through the mouth and nose is physically obstructed. Snoring, which is defined as breathing with a snorting or grunting sound while asleep, is a serious problem with a range of significant social and medical consequences.

Snoring is the sound produced by vibrating structures of the upper airway, typically during inhalation. Any membranous part of the airway lacking cartilaginous support, including the tongue, soft palate, uvula, tonsillar pillars and pharyngeal walls can vibrate. During sleep, muscle tone throughout the body decreases, and these hypotonic effects are typically exacerbated by alcohol and many prescription sleep drugs. Turbulent airflow through the hypotonic airway structures causes the harsh vibratory noise we know as snoring.

Airflow can be obstructed by a combination of factors, including:
- Obstructed nasal airways: Some people snore only during allergy seasons or when they have a sinus infection. Deformities of the nose such as a deviated septum (a structural change in the wall that separates one nostril from the other) or nasal polyps can also cause obstruction.
- Poor muscle tone in the throat and tongue: Throat and tongue muscles can be too relaxed, which allows them to collapse and fall back into the airway. This can result from deep sleep, alcohol consumption, and use of some sleeping medications which may also act as muscle relaxants. Normal aging causes further relaxation of these muscles.
- Bulky throat tissue: People who are overweight can suffer from bulky throat tissue, and it appears that children with large tonsils and adenoids can often snore.
- Long soft palate and/or uvula: A long soft palate or a long uvula (the dangling tissue in back of the mouth) can narrow the opening from the nose to the throat. When these structures vibrate and bump against one another the airway becomes obstructed, causing snoring.

Some estimates indicate that more than 45% of adult men and 30% of adult women suffer from snoring, which is often exacerbated by other breathing issues such as those caused by the common cold or influenza. Some snorers may not appear to have substantial symptoms or health issues and only become aware that they snore because of the feedback they receive from their sleep-deprived bed partner. Although the person who snores may be unaware of the problem, the effect on the sleep partner can be drastic, with frequent waking resulting in next day drowsiness and a lack of restorative sleep function. Aside from being unfair to the non-snoring partner, if the issue is not dealt with, snoring can lead to a loss of intimacy and a strains in a relationship.

For many snorers, the direct consequences to their health and well-being can be quite significant. Symptoms resulting from snoring can include being repeatedly awoken from sleep and they will suffer from a range of issues derived from sleep deprivation, including morning headaches, fatigue, irritability, poor work performance, decreased libido, weight gain and potential for anxiety and depression as well as having increased risk of developing sleep apnea, diabetes, hypertension and cardiovascular disease.

Many severe snorers may have or will eventually develop obstructive sleep apnea. A number of factors lead to narrowing of the airway and/or increase airflow turbulence can predispose to snoring; these include:
- Sleeping on the back (tongue falls backward)
- Alcohol (muscle relaxant)
- Sedatives, anti-depressants (muscle relaxants)
- Obesity (especially fat deposits around neck)
- Aging (increased laxity of soft tissues)
- Tongue falling backwards
all of which can lead to a narrowed airway. Other causes include:
- Nasal obstruction (infection, polyps, deviated septum → increased mouth breathing)
- Mouth breathing
- Increased airflow turbulence

Mouth breathing is a major contributing factor in many individuals who suffer from snoring. Mouth breathing causes an increase in airflow turbulence (compared to nasal breathing) and narrowing of the airway due to posterior migration of the base of the tongue. Both of these factors contribute to and exacerbate the severity of snoring. Mouth breathing also predisposes to dryness of the lips and mouth, halitosis, mouth ulcers, post nasal drip, dental conditions and facial deformities. In addition to being a significant cause of snoring, mouth breathing also undermines the important health benefits provided by nasal breathing. Nasal breathing enables air to flow into the nasal canal and allows the paranasal sinuses to filter, moisturize, warm and dehumidify inhaled air prior to its entry into the lungs. These important functions of the sinuses help to fight infection and ensure that the lungs receive an infusion of high quality air.

A number of problems can compromise nasal breathing including upper respiratory infections (i.e. colds, sinusitis), allergies, nasal polyps and a deviated nasal septum. Chronic impairment of nasal breathing can significantly contribute to snoring as well as predisposing to chronic sinusitis and having potentially adverse consequences for pulmonary function including the development of and/or exacerbation of asthma.

A myriad of approaches have been used in attempts to treat snoring. These include eliminating sources of nasal obstruction, the use of nasal rinses, natural remedies such as herbs, acupressure, or acupuncture, the use of antidepressants or other drug treatments, losing weight, stopping smoking, limiting alcohol and sedative use prior to sleeping, avoiding sleeping on the back, the application of splints or strips to the soft palate, teeth or nose, the use of dental devices to seal the lips, maintain closure of the jaws and prevent posterior migration of the tongue, surgical alteration of the soft tissues in the nasal canal, throat and upper airway and the use of a continuous positive airway pressure machine.

Studies have suggested that snoring alone, even without conventional sleep apnoea or hypopnoea, may disrupt sleep and produce substantial daytime hypersomnolence (Stradling, J. R. Crosby, J.H.; Payne, C.D. Self-reported snoring and daytime sleepiness in men aged 35-65 years. Thorax, 1991, 46, 807-810). 850 men, aged 35-65 years were asked a range of questions potentially related to sleepiness, snoring, and sleep apnea. The relationship between snoring and sleepiness, with allowance made for potentially confounding variables, including sleep apnea, was assessed. Positive answers to all questions about sleepiness were correlated significantly with self-reported snoring. In this study population, 17% claimed to be "often" snorers. Had they not been snorers, only 2.1% would have been expected to admit to having almost had more than one accident while driving due to sleepiness, whereas 9.9% actually did so. This means that around 7.8% of 17%, or 1.3% overall of men aged 35-65 years are perhaps at risk of having potentially fatal car accidents through snoring induced hypersomnolence. A successful treatment for snoring is may well have significant public safety advantages.

A form of serotonin (5-HT)-dependent synaptic plasticity in hypoglossal (XII) motoneurons, which control tongue muscles affecting upper airway function, that is metamodulated by metabotropic glutamate receptors has been identified. The authors propose that loss of activity in XII motoneurons is common during sleep causing snoring and, in serious cases, airway obstruction that interrupts breathing, a condition known as obstructive sleep apnea, and that their results may provide the basis for rationale development of therapeutics for obstructive sleep apnea in humans (Bocchiaro, C.M.; Feldman, J.L. Synaptic activity-independent persistent plasticity in endogenously active mammalian motoneurons. Proceedings of the National Academy of Sciences of the United States of America, 2004, 101, 4292-4295).

L-Tryptophan is an essential amino acid, meaning that it cannot be synthesized by the human body and therefore must be part of our diet. Tryptophan functions as a biochemical precursor for serotonin and melatonin, and dosing away from meals is particularly effective given the nature of its transport into the CNS.

Pharmacotherapy for Obstructive Sleep Apnea (OSA) has met with limited success; drugs evaluated include ventilator drive stimulants, CNS stimulants, antidepressants, serotonin reuptake inhibitors or antagonists and antihypertensive agents (Hudgel, D.W. and Sitthep, T. Pharmacologic treatment of sleep disordered breathing. Am. J. Resp. Crit. Care Med., 1998, 158, 691-699). Some authors (Veasey, S.C., Fenik, P., Panckeri, K., Pack, A.I. and Hendricks, J.C. The Effects of Trazodone with L-Tryptophan on Sleep disordered Breathing in the English Bulldog, Am J. Resp. Crit. Care Med., 1999, 160, 1659-1667; Ogasa, T., Ray, A. D., Michlin, C. P., Farkas, G. A. et al., Systemic Administration of Serotonin 2A/2C Agonist Improves Upper Airway Stability in Zucker Rats. Am. J. Respir. Crit. Care Med., 2004, 170, 804-810} indicate that a more rational and effective therapy, would be to activate motor neurons which innervate the dilatory muscles of the pharynx, the part of the throat situated immediately below the mouth and nasal cavity. These muscles are tonically activated to hold air passages open, reducing the likelihood of breathing problems during sleep.

Brain neurons utilizing serotonin as neurotransmitter provide excitatory input to the pharyngeal motor neurons innervating the pharyngeal muscles, and increased serotonergic input to the brainstem enhances the activity of these motor neurons. This leads to dilation of the pharynx and relief of the symptoms of OSA in an animal model (Veasey, S.C. Serotonin agonists and antagonists in obstructive sleep apnea: therapeutic potential. Am. J. Respir. Med., 2003, 2, 21-29; Ogasa *et al.,* 2004). A serotonergic treatment has also yielded positive results in human studies (Carley, D.W., Olopade, C., Ruigt, G.S. and Radulovacki, M. Efficacy of Mirtazapine in Obstructive Sleep Apnea Syndrome. Sleep, 2007, 30, 35-41).

Given the deleterious effects on sleep and quality of life for both suffers of sleep-related respiratory disorders and their partners and families, there is a major unmet need for new treatments for this class of sleep and respiratory disorders. This is particularly the case for snoring.

Doxylamine is a preferred member of the ethanolamine class of antihistamine drugs since it possesses an anti-allergy effect in human subjects superior to almost every other antihistamine on the market, with the possible exception of diphenhydramine. It is also the most effective sedative available in general sale the United States, and is seen as more sedating than some prescription hypnotics. One study reputedly found that doxylamine succinate was more effective than the barbiturate phenobarbital for use as a sedative (http://www.drugbank.ca/drugs/DB00366)

PCT Int. Appl. WO 02/056876 discloses a composition in the form of a nasal spray for the treatment of snoring comprising the antihistamine drug azelastine.

U.S. Pat. US 6,258,814 B1 discloses a composition for the treatment of sleep disorders, including snoring and sleep apnoea, comprising the antihistamine drug cetirizine.

PCT Int. Appl. WO 2007/092333 discloses the use of the anti-H1 drug olopatadine for the treatment of sleep apnoea syndrome selected from central, obstructive and mixed sleep apnoea alone or in coadministration with a melatonin agonist.

U.S. Pat. Appl. US 2006/0130833 A1 discloses the treatment of obstructive sleep apnoea and snoring with diphenydramine.

U.S. Pat. Appl. US 2004/0157813 A1 discloses that snoring patients suffer substantial lowering of oxygen-gas saturation in arterial blood (SpO2) during the breathing interruptions, with a desaturation level of 40% from the normal 90%. When melatonin is administered to patients with obstructive sleeping apnoea, a distinct effect on the sleeping registration of the patients was observed in terms of reduced number of desaturations and of SpO2 reductions below 90%.

PCT Int. Appl. WO 1998/051309 includes compositions and methods for alleviating or preventing a disordered breathing episode. The composition of the invention comprises a serotonin re-uptake inhibitor and an agent selected from the group consisting of a serotonin precursor and a serotonin agonist.

PCT Int. Appl. WO 1999/043319 discloses pharmacological methods for the prevention or amelioration of sleep-related breathing disorders *via* administration of agents or combinations of agents that possess serotonin-related pharmacological activity.

PCT Int. Appl. WO 2000/056314A1 claims compounds related to sibutramine or a pharmaceutically acceptable salt thereof (e.g. *N,N*-dimethyl-1-[1-(4-chlorophenyl)cyclobutyl]-3-methylbutylamine-HCI, optionally in the form of its monohydrate) are useful for treating sleeping disorders, including sleep apnea and snoring.

U.S. Pat. Appl. Publ. US 20060241164 discloses methods for the prevention or amelioration of sleep-related breathing disorders *via* administration of agents or combinations of agents that possess serotonin-related pharmacological activity.

PCT Int. Appl. WO 2005/063297 relates to pharmaceutical compositions, in particular controlled-release oral dosage forms, comprising a sedative agent, and melatonin or a melatonin analog. In a preferred embodiment, the sedative agent is eszopiclone.

JP 2005320254 claims certain combinations of antihistamines, for example diphenhydramine and melatonin as hypnotics.

PCT Int. Appl. WO 2005/123074 discloses a method is disclosed for the treatment of sleep disorders. The method involves administration of triprolidine, in combination with at least one further active pharmaceutical agent, for enabling an individual to wake refreshed after sleep and the method of treating such an individual with triprolidine. Use of triprolidine, in combination with at least one further active pharmaceutical agent, as active ingredient in the manufacture of a composition for the treatment of sleep disorders is also described.

U.S. Patent Appl. Publ. (2006), US 20060241164 discloses methods for the prevention or amelioration of sleep-related breathing disorders *via* administration of agents or combinations of agents that possess serotonin-related pharmacological activity.

PCT Int. Appl. (2006), WO 2006069030 claims pharmaceutical composition for the pharmacological treatment of breathing disorders and, more specifically, to compositions containing agents having serotonin receptor modulating activity for the alleviation of sleep apnea (central and obstructive) and other sleep-related breathing disorders wherein the active ingredients are released such as to extend effective blood plasma concentrations across the period of sleep.

PCT Int. Appl. WO 2007/020337 relates to the combination of: a short-acting hypnotic agent which is selected from among a modulator of receptors GABA-A, a benzodiazepine, a phenothiazine, a melatonin derivative and a melatonin receptor agonist; and a long-acting hypnotic agent which is selected from among a modulator of receptors GABA-A, a benzodiazepine, an antagonist of receptors 5HT_{2A} and a calcium ion modulator, for the treatment of sleep disorders.

PCT Int. Appl. WO 2012/170883 discloses novel pharmaceutical compositions for the treatment of sleep disorders and the induction of restorative sleep function comprising a mixture of an antihistamine drug and at least one dietary supplement. The compositions typically comprise a sedating antihistamine and selected indole-based natural products such as L-tryptophan, 5-hydroxytryptophan and melatonin, along with pharmaceutically acceptable calcium and magnesium salts and selected B vitamins.

CN 102166225 relates to a medical liquid that contains tween 80 1-3, glycerol 5-15, sodium chloride 0.5-1.5, sodium edetate 0.3-0.8, potassium sorbate 0.1-0.3 and purified water 80-90 wt.%. The medical liquid used via dripping into nasal cavity for lubricating and softening pharyngeal mucosa, keeping the nasal cavity and laryngopharyngeal mucosa wet and reducing resistance of the upper respiratory tract, and relieving snoring symptoms.

PCT Int. Appl. WO 2012/103398 relates to methods for treating obstructive sleep apnea (OSA), alleviating a negative OSA symptom, reducing snoring, or improving quality of life in a subject, comprising administering to the subject an effective amount of a composition comprising pyridostigmine before sleep.

CN 102805822 relates to a health-care product capable of relieving snoring. The product comprises Glycyrrhiza uralensis 4-8, Atractylodes lancea 15-25, Alisma orientalis 25-35, Salvia miltiorrhiza 15-25, wheat 7-15, oyster 25-45, jujube 15-35, Poria cocos polysaccharides 1-3, and Ginkgo biloba total flavone 1-3 wt. part.

### SUMMARY OF THE INVENTION

The present invention is directed to combinations of a sedating antihistamine dug comprising Doxylamine or a pharmaceutically-acceptable salt thereof, and at least one, and preferably two or more dietary supplements, comprising an indole-based compound selected from tryptophan, 5-hydroxytryptophan, and melatonin, in relation to their use for treating and thereby improving the health and quality of life of those suffering from sleep-related respiratory disorders, characterized by abnormal breathing during sleep. These include snoring, sleep disordered breathing, sleep apneas, upper airway resistance syndrome, obstructive sleep apnea, central sleep apnea and obesity hypoventilation syndrome and associated ailments.

The present invention is directed in part to the use of indole-based dietary supplements in combination with antihistamines according to the claims to provide an enhanced effect in the treatment of sleep-related respiratory disorders. The dietary supplements with utility in such combinations are *L-*tryptophan, 5-hydroxytryptophan, and melatonin.

### DETAILED DESCRIPTION

The invention is defined by the claims. Any subject-matter falling outside the scope of the claims is provided for information only. Any reference in the description to methods of treatment refer to the compounds, pharmaceutical compositions or medicaments of the present invention for use in the treatment of the human or animal body.

It has been discovered that specific combinations of antihistamines exemplified herein, with L-tryptophan, melatonin has a number of beneficial effects, particularly in the regulation of sleep. Studies of melatonin have led to the idea that melatonin is an internal sleep "facilitator" in humans, and therefore useful in the treatment of insomnia and the readjustment of circadian rhythms. There is evidence that administration of melatonin is able: (i) to induce sleep when the homeostatic drive to sleep is insufficient; (ii) to inhibit the drive for wakefulness emanating from the circadian pacemaker; and (iii) induce phase shifts in the circadian clock such that the circadian phase of increased sleep propensity occurs at a new, desired time. Therefore, exogenous melatonin can act as soporific agent, a chronohypnotic, and/or a chronobiotic, and role of melatonin in the regulation of sleep, and the use of exogenous melatonin to treat **sleep or circadian rhythm disorders is described (**Cajochen, C.; Krauchi, K.; Wirz-Justice, A. Role of melatonin in the regulation of human circadian rhythms and sleep. Journal of Neuroendocrinology, 2003, 15, 432-437). The use of melatonin in combination with antihistamines will therefore provide an improved sleep-aid.

Melatonin has other documented health benefits, having been studied in the treatment of cancer, immune disorders, cardiovascular diseases, depression, seasonal affective disorder (SAD), and sexual dysfunction. It has potent antioxidant properties. L-Tryptophan is an essential amino acid, meaning that it cannot be synthesized by the human body and therefore must be part of our diet. Amino acids, including tryptophan, act as building blocks in protein biosynthesis and in addition, tryptophan functions as a biochemical precursor for serotonin, and in turn, melatonin. Serotonin has been implicated in the regulation of sleep, depression, anxiety, appetite, sexual behavior, and body temperature.

In recent years, research has illustrated the utility of L-tryptophan's to treat insomnia. One study found that tryptophan depletion contributed to insomnia; 15 subjects suffering from insomnia were dosed with an amino acid drink that depleted tryptophan, and the participants' sleep patterns were studied. It was found that that sleep was significantly disrupted after tryptophan levels were lowered (Riemann, D.; Feige, B.; Hornyak, M.; Koch, S.; Hohagen, F.; Voderholzer, U. The tryptophan depletion test: impact on sleep in primary insomnia - a pilot study. Psychiatry Research, 2002, 109, 129-135).

5-Hydroxytryptophan (5-HTP) is a metabolite of tryptophan. In a further embodiment of this invention the indole-based dietary supplement is 5-hydroxytryptophan and in combination with antihistamines finds utility in the treatment of sleep disordered breathing.

The methods of the present invention comprise orally administering (i.e., through ingestion) a composition of the present invention to a mammal, preferably a human, to provide various health benefits, including treatment of abnormal breathing during sleep, including snoring, sleep disordered breathing, sleep apneas, upper airway resistance syndrome, obstructive sleep apnea, central sleep apnea, catathrenia and obesity hypoventilation syndrome and combinations thereof.

The compositions of the present invention are most preferably ingested by consumers primarily desiring to treatment of abnormal breathing during sleep while taking advantage of the restorative actions of the mammalian body during rest sleep. The compositions of this invention may also be ingested as a supplement to normal dietetic requirements. Frequency of administration is not limited. However, such administration for treatment of abnormal breathing during sleep is typically at least once weekly, more preferably at least 3 times weekly, and most preferably at least once daily around bedtime.

As used herein, the term "orally administering" with respect to the mammal (preferably, human) means that the mammal ingests or is directed to ingest (preferably, for the purpose of providing one or more of the health benefits described herein) one or more compositions of the present invention. In one embodiment, the composition is formulated as a tablet, capsule, food or beverage composition. Wherein the mammal is directed to ingest one or more of the compositions, such direction may be that which instructs and/or informs the user that use of the composition may and/or will provide one or more general health and/or general physiological benefits including, but not limited to, treatment of abnormal breathing during sleep.

The following are non-limiting examples of Methods which can be utilized to provide tablet formulations of the novel combinations which are useful in providing a medicament for improving abnormal breathing during sleep, treating snoring and other sleep-related problems. in mammals, especially humans.

### Method A

A tablet formulation is prepared for use in treating disorders involving abnormal breathing during sleep in mammals comprising 0.001 g to 0.05 g of a suitable sedating antihistamine, for example doxylamine as its pharmaceutically acceptable salt, for example its succinate salt form, 0.0001 g to 0.01 g of melatonin, 0.001 g to 1.0 g of L-tryptophan, 0.01 g to 0.05 g of niacin, 0.001 g to 0.01 g of pyridoxine, 0.01 to 1.0 g of a suitable mineral salt of calcium, for example calcium citrate and 0.005 to 0.5 g of a magnesium compound such as magnesium oxide. Suitable excipients for this tablet formulation include dicalcium phosphate, acceptable dyes such as FD&C Blue #1 aluminum lake, magnesium stearate, microcrystalline cellulose and sodium starch glycolate. The tablet is dosed to human subjects in order to improve their health, especially in the treatment of sleep problems, including abnormal breathing during sleep, snoring and other sleep-related problems.

### Method B

A tablet formulation is prepared for use in treating disorders involving abnormal breathing during sleep in mammals comprising 0.001 g to 0.05 g of a suitable sedating antihistamine, for example doxylamine as its pharmaceutically acceptable salt, for example its succinate salt form, 0.0001 g to 0.01 g of melatonin, 0.001 g to 1.0 g of L-tryptophan, 0.01 g to 0.05 g of niacin and 0.001 g to 0.01 g of pyridoxine. Suitable excipients for this tablet formulation include dicalcium phosphate, calcium citrate, acceptable dyes such as FD&C Blue #1 aluminum lake, magnesium stearate, microcrystalline cellulose and sodium starch glycolate. The tablet is dosed to human subjects in order to improve their health, especially in the treatment of sleep problems, including abnormal breathing during sleep, snoring and other sleep-related problems.

### Method C

A tablet formulation is prepared for use in treating disorders involving abnormal breathing during sleep in mammals comprising 0.001 g to 0.05 g of a suitable sedating antihistamine, for example doxylamine as its pharmaceutically acceptable salt, for example its succinate salt form, 0.0001 g to 0.01 g of melatonin, 0.001 g to 1.0 g of L-tryptophan, 0.01 g to 0.05 g of niacin and 0.01 g to 0.1 g of theanine. Suitable excipients for this tablet formulation include dicalcium phosphate, calcium citrate, acceptable dyes such as FD&C Blue #1 aluminum lake, magnesium stearate, microcrystalline cellulose and sodium starch glycolate. The tablet is dosed to human subjects in order to improve their health, especially in the treatment of sleep problems, including abnormal breathing during sleep, snoring and other sleep-related problems.

### Method D

A tablet formulation is prepared for use in treating disorders involving abnormal breathing during sleep in mammals comprising 0.005 g to 0.01 g of a suitable sedating antihistamine, for example diphenhydramine as its pharmaceutically acceptable salt, for example its hydrochloride or citrate salt form, 0.0001 g to 0.01 g of melatonin, 0.001 g to 1.0 g of L-tryptophan, 0.01 g to 0.05 g of niacin, 0.001 g to 0.01 g of pyridoxine, 0.01 to 1.0 g of a suitable mineral salt of calcium, for example calcium citrate and 0.005 to 0.5 g of a magnesium compound such as magnesium oxide. Suitable excipients for this tablet formulation include dicalcium phosphate, acceptable dyes such as FD&C Blue #1 aluminum lake, magnesium stearate, microcrystalline cellulose and sodium starch glycolate. The tablet is dosed to human subjects in order to improve their health, especially in the treatment of sleep problems, including abnormal breathing during sleep, snoring and other sleep-related problems.

### Method E

A tablet formulation is prepared for use in treating disorders involving abnormal breathing during sleep in mammals comprising 0.05 g to 0.1 g of a suitable sedating antihistamine, for example promethazine as its pharmaceutically acceptable salt, for example its hydrochloride salt form, 0.0001 g to 0.01 g of melatonin, 0.001 g to 1.0 g of L-tryptophan, 0.01 g to 0.05 g of niacin, 0.001 g to 0.01 g of pyridoxine, 0.01 to 1.0 g of a suitable mineral salt of calcium, for example calcium citrate and 0.005 to 0.5 g of a magnesium compound such as magnesium oxide. Suitable excipients for this tablet formulation include dicalcium phosphate, acceptable dyes such as FD&C Blue #1 aluminum lake, magnesium stearate, microcrystalline cellulose and sodium starch glycolate. The tablet is dosed to human subjects in order to improve their health, especially in the treatment of sleep problems, including abnormal breathing during sleep, snoring and other sleep-related problems.

### Method F

A composition of 0.025 g doxylamine succinate, and the indole-based dietary supplements L-tryptophan (0.5 g) melatonin (0.005 g) were dissolved in pasteurized milk (250 mL) or a suitable fruit juice such as mango juice (250 mL), or a combination of juices. Niacin (0.25 g), vitamin B6 (0.1 g) and calcium citrate (2.5 g) were added, followed by a source of carbohydrate, with vitamin C (0.5 g) and a suitable approved flavoring. This formulation is in a beverage form as a drink dosed to human subjects at bedtime in order to improve their health, especially in the treatment of sleep problems, including abnormal breathing during sleep, snoring and other sleep-related problems. The beverage form can be utilized to improve sleep, snoring and other sleep-related problems before, during or after a long aircraft flight.

The following are non-limiting examples of the present compositions which are prepared utilizing conventional methods. The following examples are provided to illustrate the invention and are not intended to limit the scope thereof in any manner.

### Example 1

Doxylamine succinate (0.80 g), L-tryptophan (32.50 g), melatonin (0.15 g), niacin (0.50 g) and pyridoxine (0.05 g) were all weighed on a Ohaus Explorer scale and combined with microcrystalline cellulose NF105 (Avicel™) (1.0 g). The ingredients were mixed thoroughly in a sterilized plastic container and the mixture was now ready for the capsule preparation procedure.

Utilizing a Jaansun® Capsule Machine 100, the mixture was carefully placed into #0 Clear Locking gelatin capsules (part # 30-1988-5000EA, obtained from PCCA USA, 9901 S. Wilcrest Drive, Houston, TX 77099-5132) as described below, following the manufacturer's instructions closely.

The loader filled with empty capsules was placed on top of the separator plate so that it could slide freely from left to right. The loader was positioned on the blue rails and slid to the left or right till it stopped against one set of the blue posts. The white plate was then gently pushed into the spring block, and the capsules were allowed to drop through the loader plates with the lid end up. The loader was lifted up and off the capsule machine.

One of the retainer knobs was grasped and the separator plate was shaken to drop the capsules into the machine, ensuring that the capsules were sitting in the capsule machine so that the capsule tops could not rise above the blue metal ledge located just beneath the retainer knobs. Once the capsules had dropped into the machine, the capsules were gently tapped to ensure they were all at the required height; the procedure was then repeated for the remaining 50 capsules.

The loader was lifted up and off of the capsule machine, and all capsules were tapped gently to ensure they were sitting level in the capsule machine. The retainer plate was then placed on top of the separator plate with the beveled edges facing up, parallel to each set of retaining knobs. Each retainer knob was turned so that approximately ⅓ of the knob was covering the plate, and both pinch knobs were hand-tightened at the same time. The separator plate was then lifted up and off of the machine, and checked for capsule bases. Before setting the separator plate aside, it was ensured that the black retaining knobs were facing upwards.

The black pinch knobs were loosened and the capsules were allowed to drop down into the capsule machine so they were sitting flush with the top of the white pinch plate. The black pinch knobs were lightly tightened to hold the capsule bases in place during the filling process; the optional powder dam was placed on the top of the machine and the requisite clips applied over the powder dam and the short notch under the machine.

The thoroughly-mixed ingredients, as described above, were placed in powder form in the center of the machine and spread evenly over the top of the white pinch plate with a powder scraper. The machine was gently tapped in order to move the powders back and forth on the white pinch plate. The powders were tamped with a tamper to push air out of, and powder into the capsules, then the powder was redistributed over the capsules with a scraper as needed, checking for fill uniformity. This tapping, tamping, and scraping process was repeated until all the powder was neatly packed into the capsules.

Once it appeared that the majority of the powder has been packed into the capsules, the clips and powder dam were removed, and the tamper was used to check for capsule fill uniformity. If one end of tamper was sitting higher than the other end, the powder distribution was evened out using the tamper prongs as a scoop. The capsules were now ready to be reassembled by the following procedure.

The separator plate was placed on the machine with the notches to the front door, and both pinch knobs were loosened, but with the retainer knobs holding the retainer plate in place. The depth plate was gently bounced while gradually applying pressure to raise the depth plate up. Pressure was applied to both the front and back of the plates to bring all the capsules back together.

The separator plate was then lifted up, and checked underneath to ensure that all capsule bases had been reattached to the lids; and the retainer knobs were checked again to ensure that they are still holding the retainer plate in place. The separator plate was turned over and each capsule locked by gently pressing down on each one individually. The retainer plate was removed by rotating the retainer knobs and lifting the plate up and off the separator plate. The capsules were removed from the separator plate by turning the plate over and guiding the capsules into a towel, and then the four corners of the towel were brought together and the capsules were shaken, thereby cleaning the capsules and loading the capsule lids.

The batch of 100 capsules was then available for clinical evaluation, in a typical daily dose of 1 or 2 capsules at bedtime to human subjects in order to treat of sleep problems, including abnormal breathing during sleep, snoring and other sleep-related problems, especially in subjects who take dietary supplements ensuring healthy and adequate levels of the minerals calcium and magnesium.

### Example 2

Doxylamine succinate (0.80 g), L-tryptophan (32.50 g), melatonin (0.15 g), niacin (0.50 g), pyridoxine (0.05 g), calcium citrate (0.25 g) and magnesium oxide (0.10 g) were all weighed carefully and combined with microcrystalline cellulose NF105 (Avicel™) (1.0 g). The ingredients were mixed thoroughly in a sterilized plastic container and the mixture was converted into a capsule formulation, as described in Example 1, but utilizing #0 Clear Locking gelatin capsules. The batch of 100 capsules was then available for clinical evaluation, in a typical daily dose of 2 capsules at bedtime dosed to human subjects in order to improve their health, especially in the treatment of sleep problems, including abnormal breathing during sleep, snoring and other sleep-related problems.

### Example 3

Doxylamine succinate (0.80 g), L-tryptophan (32.50 g), melatonin (0.15 g) and niacin (0.50 g) were all weighed carefully and combined with microcrystalline cellulose NF105 (Avicel™) (1.0 g). The ingredients were mixed thoroughly in a sterilized plastic container and the mixture was converted into a capsule formulation, as described in Example 1. The batch of 100 capsules was then available for clinical evaluation, in a typical daily dose of 2 capsules at bedtime in subjects suffering from sleep problems, including abnormal breathing during sleep and snoring, especially subjects who take dietary supplements ensuring healthy and adequate levels of the minerals calcium and magnesium.

### Example 4

Doxylamine succinate (0.80 g), L-tryptophan (32.50 g), melatonin (0.15 g), niacin (0.50 g), powdered calcium citrate (0.25 g) and magnesium oxide (0.10 g) were all weighed carefully and combined with microcrystalline cellulose NF105 (Avicel™) (1.0 g). The ingredients were mixed thoroughly in a sterilized plastic container and the mixture was converted into a capsule formulation, as described in Example 1. The batch of 100 capsules was then available for clinical evaluation, in a typical daily dose of 2 capsules at bedtime in subjects suffering from sleep problems, including abnormal breathing during sleep and snoring, especially subjects who take dietary supplements ensuring healthy and adequate levels of the minerals calcium and magnesium.

### Example 5 (reference example)

Diphenhydramine hydrochloride (1.60 g), L-tryptophan (32.50 g), melatonin (0.15 g), niacin (0.50 g), pyridoxine (0.05 g), calcium citrate (0.25 g) and magnesium oxide (0.10 g) were all weighed carefully and combined with microcrystalline cellulose NF105 (Avicel™) (1.0 g). The ingredients were mixed thoroughly in a sterilized plastic container and the mixture was converted into a capsule formulation, as described in Example 1. The batch of 100 capsules was then available for clinical evaluation, in a typical daily dose of 2 capsules at bedtime in subjects suffering from sleep problems, including abnormal breathing during sleep and snoring.

### Example 6

A healthy male volunteer aged 64 took one capsule, prepared as described in Example 3 (containing 8 mg doxylamine and other substances as described) regularly at bedtime during a long-haul vacation trip. His spouse repeatedly reported a highly unusual reduction of snoring the following morning. This effect was reported in writing on his return to the US.

### Example 7

A healthy male volunteer aged 58 ingested 2 capsules, prepared as described in Example 3 (representing a nightly dose of 16 mg doxylamine and other substances as described) nightly on several occasions after west-east transatlantic travel and his spouse repeatedly reported significant decreases in snoring events during the night.

Acid addition salts of the antihistamine and optional melatonin, 5-hydroxytryptophan and tryptophan combinations and other agents employed in the invention can be prepared in a conventional manner by treating a solution or suspension of the corresponding free base with one chemical equivalent of a pharmaceutically acceptable acid. Conventional concentration or crystallization techniques can be employed to isolate the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic acids such as methanesulfonic, benzene sulfonic, *p*-toluenesulfonic, and related organic or inorganic acids. The antihistamine and optional melatonin, 5-hydroxytryptophan and tryptophan combinations and their pharmaceutically acceptable salts, may be administered alone or in combination with pharmaceutically acceptable carriers, in either single or multiple doses. Suitable pharmaceutical carriers include inert solid diluents or fillers, sterile aqueous solutions, oils (e.g. peanut oil, sesame oil) and various organic solvents. Enterically coated tablets are a preferred formulation when 5-hydroxytryptophan is utilized as one of the indole-based dietary supplements. The pharmaceutical compositions formed by combining the antihistamine and optional melatonin, 5-hydroxytryptophan and tryptophan combinations and pharmaceutically acceptable carriers can be readily administered in a variety of dosage forms such as tablets, powders, lozenges, emulsions, oil soft gels, syrups, injectable solutions and the like. These pharmaceutical compositions can, if desired, contain additional ingredients such as flavorings, binders, excipients, taste-masking agents and the like. Thus, for purposes of oral administration, tablets containing various excipients such as sodium citrate, calcium carbonate and calcium phosphate may be employed along with various disintegrants such as starch, methylcellulose, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tableting purposes. Solid compositions of a similar type may also be employed as fillers in soft and hard filled gelatin capsules. Preferred materials for this include lactose or milk sugar and high molecular weight polyethylene glycols. When aqueous suspensions, elixirs or beverages are desired for oral administration, the essential active ingredient therein may be combined with a large range of various sweetening or flavoring agents, coloring matter or dyes and, if desired, emulsifying or suspending agents, together with diluents such as water, ethanol, propylene glycol, glycerin and combinations thereof.

For parenteral administration, solutions containing the antihistamine and optional melatonin, tryptophan and 5-hydroxy-tryptophan combinations or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

The effective dosages for the antihistamine, melatonin, 5-hydroxytryptophan and tryptophan combinations employed in the methods of this invention will depend on the intended route of administration and factors such as the age and weight of the patient. The dosages will also depend on the particular condition to be treated and will generally range from about 0.1 to about 300 mg/kg body weight of the patient per day, with administration carried out in single or divided dosages.

Unless defined otherwise, all technical and scientific terms used herein have the same meanings as commonly understood by one of ordinary skill in the art. Unique expressions used in the text are appropriately defined.

## Claims

1. A pharmaceutical composition comprising a mixture of:
an antihistamine drug comprising Doxylamine or a pharmaceutically-acceptable salt thereof; and
at least one dietary supplement comprising an indole-based compound selected from L-tryptophan, 5-hydroxytryptophan and melatonin or a pharmaceutically acceptable salt thereof;
for use in the treatment, prevention or amelioration of a sleep-related breathing or respiratory disorder.

2. The composition for use according to claim 1, wherein the antihistamine drug is doxylamine succinate.

3. The composition for use according to claim 1 or claim 2, further comprising at least one vitamin and pharmaceutically acceptable minerals to enhance the effect of the indole-based compound.

4. The composition for use according to any preceding claim, further comprising a pharmaceutically acceptable calcium salt, a pharmaceutically acceptable magnesium salt, and at least one pyridine-based vitamin.

5. The composition for use according to claim 4, wherein said pyridine-based vitamin is vitamin B₆ or a pharmaceutically acceptable salt thereof.

6. A composition for use according to claim 1 comprising: doxylamine succinate; L-tryptophan or a pharmaceutically acceptable salt thereof; melatonin or a pharmaceutically acceptable salt thereof; a pharmaceutically acceptable calcium salt; a pharmaceutically acceptable magnesium salt; vitamin B₃ or a pharmaceutically acceptable salt thereof; and a pharmaceutically acceptable carrier.

7. The composition for use according to claim 6, further comprising an amino acid from a group consisting of theanine, GABA, valerian and combinations thereof.

8. The composition for use according to any preceding claim, formulated as a tablet, fast dissolving tablet, capsule, gel formulation, a liqui-gel or a beverage.

9. The composition for use according to any preceding claim, wherein the dietary supplement is in a form selected from a group consisting of a delayed release, slow release, sublingual, intranasal and transdermal formulation.

10. The composition for use according to any preceding claim, wherein the sleep-related respiratory disorder is selected from a group consisting of snoring, sleep disordered breathing, sleep apneas, upper airway resistance syndrome, obstructive sleep apnea, central sleep apnea, hypopnea, catathrenia, obesity hypoventilation syndrome, central apnea syndromes, Cheyne-Stokes respiration, Obstructive Sleep Apnea Syndrome, Upper Airway Resistance Syndrome, Alveolar hypoventilation syndrome, Pickwickian syndrome and combinations thereof.

11. The composition for use according to any preceding claim, for use in the treatment of a sleep-related breathing disorder in a non-human mammal.

## Patentansprüche

1. Eine pharmazeutische Rezeptur umfassend ein Gemisch aus:
einem Antihistaminikum umfassend Doxylamin oder ein pharmazeutisch akzeptables Salz davon; und
wenigstens einem Nahrungsergänzungsmittel umfassend eine Indol-basierte Verbindung ausgewählt aus *L*-Tryptophan, 5-Hydroxytryptophan und Melatonin oder einem pharmazeutisch akzeptablem Salz davon;
zur Verwendung in Behandlung, Vorbeugung oder Besserung einer schlafbezogenen Atmungsstörung oder Atemwegserkrankung.

2. Die Rezeptur zur Verwendung gemäß Anspruch 1, wobei das Antihistaminikum Doxylaminsuccinat ist.

3. Die Rezeptur zur Verwendung gemäß Anspruch 1 oder Anspruch 2, ferner umfassend wenigstens ein Vitamin oder pharmazeutisch akzeptables Mineral zur Steigerung des Effekts der Indol-basierten Verbindung.

4. Die Rezeptur zur Verwendung gemäß einem der voranstehenden Ansprüche, ferner umfassend ein pharmazeutisch akzeptables Kalziumsalz, ein pharmazeutisch akzeptables Magnesiumsalz und wenigstens ein Pyridin-basiertes Vitamin.

5. Die Rezeptur zur Verwendung gemäß Anspruch 4, wobei das Pyridin-basierte Vitamin Vitamin B₆ oder ein pharmazeutisch akzeptables Salz davon ist.

6. Eine Rezeptur zur Verwendung gemäß Anspruch 1 umfassend:
Doxylaminsuccinat; *L*-Troptophan oder ein pharmazeutisch akzeptables Salz davon;
Melatonin oder ein pharmazeutisch akzeptables Salz davon; ein pharmazeutisch akzeptables Kalziumsalz; ein pharmazeutisch akzeptables Magnesiumsalz; Vitamin B3 oder ein pharmazeutisch akzeptables Salz davon; und einen pharmazeutisch akzeptablen Träger.

7. Die Rezeptur zur Verwendung gemäß Anspruch 6, ferner umfassend eine Aminosäure aus einer Gruppe umfassend Theanin, γ-Aminobuttersäure, Baldrian und Kombinationen daraus.

8. Die Rezeptur zur Verwendung gemäß einem der voranstehenden Ansprüche, formuliert als eine Tablette, schnelllösliche Tablette, Kapsel, Gelformulierung, ein Flüssiggel oder ein Getränk.

9. Die Rezeptur zur Verwendung gemäß einem der voranstehenden Ansprüche, wobei das Nahrungsergänzungsmittel in einer Form ist, die ausgewählt ist aus einer Gruppe bestehend aus verzögerter Freisetzung, langsamer Freisetzung, sublinguale, intranasale oder transdermale Formulierung.

10. Die Rezeptur zur Verwendung gemäß einem der voranstehenden Ansprüche, wobei die schlafbezogene Atemwegserkrankung ausgewählt ist aus einer Gruppe bestehend aus Schnarchen, schlafbezogene Atmungsstörungen, Schlafapnoen, Upper Airway Resistance Syndrom, obstruktive Schlafapnoe, zentrale Schlafapnoe, Hypopnoe, Katathrenie, Obesitas-Hypoventilations-Syndrom, Zentralapnoesyndrome, Cheyne-Stokes-Atmung, Obstruktives Schlafapnoe-Syndrom, Upper Airway Resistance Syndrom, Alveoläres Hypoventilations-Syndrom, Pickwick-Syndrom und Kombinationen daraus.

11. Die Rezeptur zur Verwendung gemäß eeinem der voranstehenden Ansprüche zur Behandlung einer schlafbezogenen Atmungsstörung in einem nicht-menschlichen Säugetiers.

## Revendications

1. Composition pharmaceutique comprenant un mélange d' :
― un médicament antihistaminique comprenant de la Doxylamine ou un sel pharmaceutiquement acceptable de celle-ci ; et
― au moins un complément alimentaire comprenant un composé à base d'indole choisi parmi le *L*-tryptophane, le 5-hydroxytryptophane et la mélatonine ou un sel pharmaceutiquement acceptable de ceux-ci ;
pour son utilisation dans le traitement, la prévention ou l'amélioration d'un trouble respiratoire ou de la respiration lié au sommeil.

2. Composition pour son utilisation selon la revendication 1, dans laquelle le médicament antihistaminique est le succinate de doxylamine.

3. Composition pour son utilisation selon la revendication 1 ou la revendication 2, comprenant en outre au moins une vitamine et des minéraux pharmaceutiquement acceptables pour renforcer l'effet du composé à base d'indole.

4. Composition pour son utilisation selon l'une quelconque des revendications précédentes, comprenant en outre un sel de calcium pharmaceutiquement acceptable, un sel de magnésium pharmaceutiquement acceptable, et au moins une vitamine à base de pyridine.

5. Composition pour son utilisation selon la revendication 4, dans laquelle ladite vitamine à base de pyridine est la vitamine B6 ou un sel pharmaceutiquement acceptable de celle-ci.

6. Composition pour son utilisation selon la revendication 1, comprenant : du succinate de doxylamine ; du *L*-tryptophane ou un sel pharmaceutiquement acceptable de celui-ci ; de la mélatonine ou un sel pharmaceutiquement acceptable de celle-ci ; un sel de calcium pharmaceutiquement acceptable ; un sel de magnésium pharmaceutiquement acceptable ; la vitamine B₃ ou un sel pharmaceutiquement acceptable de celle-ci ; et un véhicule pharmaceutiquement acceptable.

7. Composition pour son utilisation selon la revendication 6, comprenant en outre un acide aminé d'un groupe constitué par la théanine, le GABA, la valériane et leurs combinaisons.

8. Composition pour son utilisation selon l'une quelconque des revendications précédentes, formulée sous la forme d'un comprimé, d'un comprimé à dissolution rapide, d'une capsule, d'une formulation de gel, d'un liqui-gel ou d'une boisson.

9. Composition pour son utilisation selon l'une quelconque des revendications précédentes, dans laquelle le complément alimentaire est sous une forme choisie dans un groupe constitué par une formulation à libération retardée, à libération lente, sublinguale, intranasale et transdermique.

10. Composition pour son utilisation selon l'une quelconque des revendications précédentes, dans laquelle le trouble respiratoire lié au sommeil est choisi dans un groupe constitué par le ronflement, les troubles respiratoires du sommeil, les apnées du sommeil, le syndrome de résistance des voies aériennes supérieures, l'apnée obstructive du sommeil, l'apnée centrale du sommeil, l'hypopnée, la catathrénie, le syndrome d'hypoventilation de l'obésité, les syndromes d'apnée centrale, la respiration de Cheyne-Stokes, le syndrome d'apnée obstructive du sommeil, le syndrome de résistance des voies aériennes supérieures, le syndrome d'hypoventilation alvéolaire, le syndrome de Pickwick et leurs combinaisons.

11. Composition pour son utilisation selon l'une quelconque des revendications précédentes, pour une utilisation dans le traitement d'un trouble respiratoire lié au sommeil chez un mammifère non humain.
